# EUROPÄISCHE PATENTSCHRIFT

(11) **EP 1 477 238 B1**
(45) Veröffentlichungstag und Bekanntmachung des Hinweises auf die Patenterteilung: **17.11.2010**
(21) Anmeldenummer: 04007482.5
(22) Anmeldetag: 27.03.2004
(51) Int. Cl.: B08B 9/032, A61B 19/00, A61M 25/00

(54) **Vorrichtung zum Reinigen und/oder Desinfizieren von langgestreckten Hohlkörpern, insbesondere von medizinischen Schläuchen und Kathedern**
Apparatus for cleaning and/or desinfecting elongated hollow bodies, in particular medical tubes and catheters
Appareil pour nettoyer et/ou désinfecter des corps creux allongés, en particulier tubes médicaux et catheters

(30) Priorität: 16.05.2003 DE 10321991
(43) Veröffentlichungstag der Anmeldung: 17.11.2004
(73) Patentinhaber: BHT Hygienetechnik GmbH, 83368 Gersthofen (DE)
(72) Erfinder: Annecke, Karl-Heinz, Dr., 64625 Bensheim (DE)
(74) Vertreter: von Bülow, Tam

(56) Entgegenhaltungen:
- EP-A- 0 709 056
- EP-A- 1 338 237

## Beschreibung

Die Erfindung bezieht sich auf eine Vorrichtung zum Reinigen und/oder Desinfizieren von langgestreckten Hohlkörpern, insbesondere von medizinischen schläuchen und Kathetern.

Die ältere, nicht vorveröffentlichte EP 1 338 237 A2 schlägt für die Validierung der Reinigung mehrerer Kanäle von Endoskopen vor, jedem Kanal einen Flüssigkeitsbehälter mit einem vorbestimmten Volumen zuzuordnen und zu überprüfen, ob dieses Volumen innerhalb vorgegebener Zeit durch den Kanal geflossen ist. Die Flüssigkeitsbehälter sind über eine Strömungsverengung an eine Druckluftquelle abgeschlossen. Ein an den Druckluftkreis angeschlossenen Drucksensor misst den Luftdruck und stellt bei stärkerem Druckabfall fest, dass die Flüssigkeitsbehälter leer sind.

Die EP 0 709 056 A1, der für den Gegenstand des Anspruchs 1 als nächstliegender Stand der Technik angesehen wird, beschreibt ein Verfahren und eine Vorrichtung zum Reinigen und Desinfizieren von Endoskopen, mit mindestens einer an einem Wasserkanal angeschlossenen Kupplung zur Verbindung des Strömungsrichtung vor der Kupplung ein Drucksensor zum Erfassen des im Hohlkörper entstehenden Druckes angeordnet ist.

Die EP 0 711 529 A1 beschreibt ein Verfahren zum Prüfen und Reinigen von Endoskopen, bei der jeder Fluidkanal über einen Verteiler mit Ventilen an eine Fluidquelle anschließbar ist. Mittels eines Durchflußprüfgerätes wird die Durchlässigkeit der Kanäle einzeln oder in Gruppen nacheinander geprüft, indem den Kanälen Fluid aus der Fluidquelle zugeführt wird. Zur Überprüfung der Durchlässigkeit wird überprüft, ob ein bestimmtes Fluidvolumen in einer bestimmten Zeiteinheit durch den jeweiligen Kanal geleitet wird. Die Messung der Durchflußmenge pro Zeiteinheit erfolgt dadurch, daß das Fluid unter Druck durch den Kanal gepresst wird und der zeitliche Verlauf des Druckabfalles ausgewertet wird, woraus sich das durchströmte Volumen bestimmen läßt. Aufgrund des ermittelten Volumens pro Zeiteinheit soll dann das spezifische Endoskop identifiziert werden, indem der gemessene Wert mit gerätespezifischen Werten verglichen wird.

Die DE 296 20 011 U1 schlägt vor, zum Reinigen mehrerer Katheter oder Schläuche an einem Wasserkanal mehrere Aufsteckdüsen anzubringen, die eine unlösbar mit ihr verbundene Befestigungseinrichtung aufweisen, die die Außenseite des zu reinigenden Gegenstandes form- oder kraftschlüssig halten. Diese Aufsteckdüsen dienen als Kupplung zur Verbindung des Schlauches mit dem Wasserkanal.

Zur Validierung des Reinigungs- und/oder Desinfektionsergebnisses von langen dünnen Hohlkörpern wird die Forderung gestellt, festzustellen, ob tatsächlich die gewünschte Menge Flüssigkeit durch den Hohlkörper geflossen ist.

Die DE 36 01 395 A1 beschreibt eine Vorrichtung zur Anzeige anormaler Betriebszustände bei einer Endoskopwaschmaschine.

Mit einer Vielzahl von Sensoren werden bestimmte Parameter, wie z.B. Pegelstände von Reinigungsflüssigkeit, gemessen bzw. überwacht und daraus ermittelt, ob eine Störung oder ein anormaler Betriebszustand vorliegen.

Aufgabe der Erfindung ist es daher, die bekannte Vorrichtung dahingehend zu verbessern, daß mit vereinfachtem technischem Aufwand überprüft werden kann, ob ein Hohlkörper gereinigt wurde.

Diese Aufgabe wird durch die im Patentanspruch 1 angegebenen Merkmale gelöst. Vorteilhafte Ausgestaltungen und Weiterbildungen der Erfindung sind den Unteransprüchen zu entnehmen.

Das Grundprinzip der Erfindung besteht darin, im Wasserkanal vor der Kupplung zum Hohlkörper eine Strömungsverengung und einen Drucksensor mit Speicherfunktion anzuordnen, wobei der Drucksensor aus einer an den Wasserkanal angeschlossenen Hülse und einem in der Hülse verschieblichen Kolben besteht. Durch die Strömungsverengung wird der Speisedruck der Reinigungsflüssigkeit zum Wasserkanal bei einwandfreiem Durchfluß durch den Hohlkörper herabgesetzt. Ist der Hohlkörper dagegen ganz oder teilweise verstopft, so entsteht ein demgegenüber erhöhter Staudruck, der vom Drucksensor erfaßt und gespeichert wird. Nach Beendigung des Reinigungsvorganges kann an diesem gespeicherten Druckwert erkannt werden, daß der entsprechende Hohlkörper nicht einwandfrei gereinigt wurde.

Der Drucksensor muß nach einer vorteilhaften Weiterbildung der Erfindung lediglich messen und speichern, ob der gemessene Druck einen vorbestimmten Wert überschritten hat. Der Drucksensor kann damit quasi als Schalter ausgeführt sein, der nur die zwei Zustände "vorgegebener Druck überschritten" oder "vorgegebener Druck nicht überschritten" misst und speichert.

Nach einer besonders vorteilhaften Ausgestaltung der Erfindung wird der Kolben durch sein Eigengewicht in Richtung zum Wasserkanal gedrückt wird. Ist der vorbestimmte Druck überschritten, so wird dieser Kolben vollständig aus der Hülse herausgedrückt. Die Speicherfunktion liegt dann darin, daß der Kolben nicht mehr in der Hülse ist. Ein wesentlicher Vorteil liegt noch darin, daß dann die an den Wasserkanal angeschlossene Hülse als Überdruckventil wirkt, womit erreicht wird, daß der Druck in einem ganz oder teilweise verstopften Hohlkörper einen bestimmten Grenzwert nicht überschreitet. Der Kolben ist durch ein elastisches Halteorgan unverlierbar an dem Wasserkanal oder der Hülse befestigt. Es kann sich hierbei um eine einfache seilartige Befestigung handeln.

Nach einer anderen Variante kann der Kolben auch durch eine Feder in Richtung auf den Wasserkanal vorgespannt sein, wobei dann Einrichtungen vorgesehen sind, um den Kolben in einer vorbestimmten oberen Stellung zu halten, auch wenn der Druck später wieder absinkt, wodurch die Speicherfunktion erhalten wird. Diese Einrichtung kann beispielsweise darin bestehen, daß an dem Kolben eine Rastnut vorhanden ist und an der Hülse eine Feder angebracht ist, die bei Erreichen einer vorbestimmten Grenzstellung in die Rastnut einrastet. Eine andere Möglichkeit besteht darin, am Kolben und der Hülse jeweils Magneten anzubringen, die den Kolben ebenfalls in einer vorbestimmten Stellung halten, sofern diese durch den gemessenen Druck erreicht wurde. Nach Abschluß des Reinigungsvorganges kann man dann ebenfalls sehr einfach erkennen, ob einer der Kolben in der oberen Grenzstellung gehalten ist, was signalisiert, daß das Reinigungsergebnis nicht zufriedenstellend war.

Nach einer vorteilhaften Weiterbildung der Erfindung ist die Strömungsverengung, die in Strömungsrichtung vor dem Drucksensor liegt, als einstellbare Düse ausgebildet, die im einfachsten Falle aus einer in den Wasserkanal einschraubbaren Schraube besteht. Hiermit kann eine Anpassung an den Speisedruck zum Reinigungskanal und an den gewünschten Druck der durch den Hohlkörper strömenden Flüssigkeit vorgenommen werden. Auch kann der Ansprechdruck des Sensors eingestellt werden, im einfachsten Fall des durch sein Eigengewicht belasteten Kolbens durch das Gewicht des Kolbens, bei Verwendung einer Feder durch Justieren der Feder oder Auswechseln der Feder durch eine Feder mit anderer Federhärte.

Im folgenden wird die Erfindung anhand von Ausführungsbeispielen im Zusammenhang mit der Zeichnung ausführlicher erläutert. Es zeigt:
- Fig. 1: eine Prinzipskizze einer Vorrichtung nach der Erfindung;
- Fig. 2: ein bevorzugtes Ausführungsbeispiel einer bei der Erfindung verwendeten Strömungsverengung und eines Drucksensors mit Speicherfünktion;
- Fig. 3: ein zweites Ausführungsbeispiel eines Drucksensors mit Speicherfunktion; und
- Fig. 4: ein drittes Ausführungsbeispiel eines Drucksensors mit Speicherfunktion.

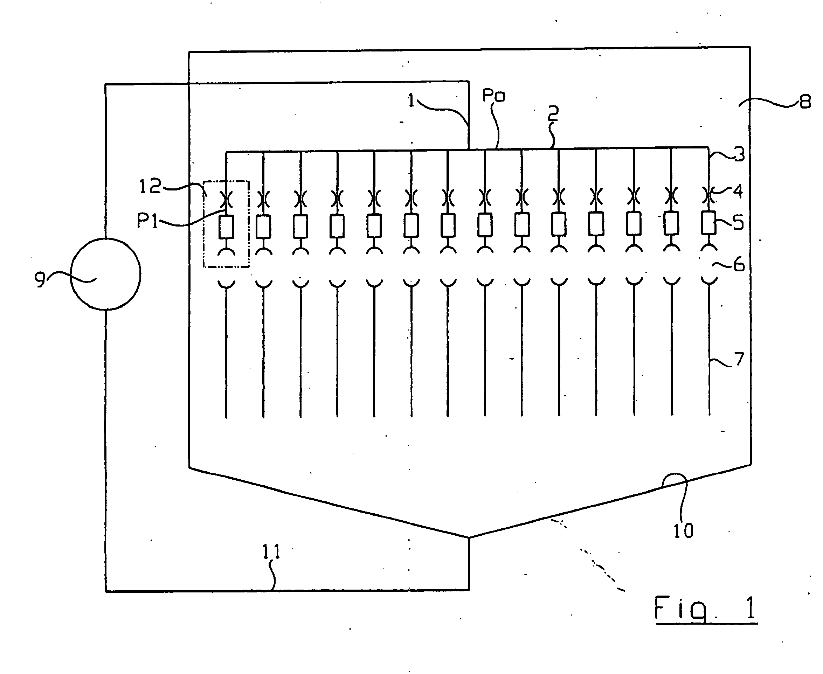

Fig. 1 zeigt einen Wasserzulauf 1, der über einen Rohrverteiler 2 zu einer Vielzahl von Wasserkanälen 3 verzweigt. In jedem Wasserkanal 3 ist eine einstellbare Strömungsverengung 4, ein Drucksensor 5 mit Speicherfunktion und eine Kupplung 6 vorhanden, wobei über jede Kupplung ein zu reinigender Hohlkörper 7, insbesondere ein medizinischer Schlauch oder ein Katheter anschließbar ist. Die genannten Gegenstände befinden sich in einem Waschraum 8 einer Reinigungs- und Desinfektionsmaschine, bei welcher Reinigungsflüssigkeit durch eine Umwälzpumpe 9 vom Sumpf 10 der Maschine über einen Rückführkanal 11 zum Wasserzulauf 1 gefördert wird. Die übrigen Teile einer handelsüblichen Reinigungs- und Desinfektionsmaschine, wie z.B. Frischwasserzulauf, Steuereinrichtung, Dosiereinrichtung für Reinigungs- und Desinfektionsmittel etc., sind der Übersichtlichkeit halber fortgelassen.

Die Strömungsverengung 4, der Drucksensor 5 und die Kupplung 6 können als ein zusammenhängendes Modul 12 ausgebildet und an den jeweiligen Wasserkanal 3 angeschlossen sein. Die Kupplung 6 kann selbstschließend ausgebildet sein derart, daß sie bei nicht angeschlossenem Hohlkörper 7 geschlossen ist und durch Anschließen eines Hohlkörpers automatisch öffnet. Es ist aber auch möglich, jedem Modul 12 noch ein nicht dargestelltes Absperrventil zuzuordnen. Die Kupplung 6 kann - wie dargestellt - ein sog. Luer-Anschluß sein.

Die Arbeitsweise der Vorrichtung ist wie folgt:
Es wird davon ausgegangen, daß an alle dargestellten Module 12 je ein Hohlkörper angeschlossen ist. Durch die Umwälzpumpe 9 wird Flüssigkeit mit einem Eingangsdruck P₀ über den Rohrverteiler 2 den einzelnen Wasserkanälen 3 zugeführt. Dieser Eingangsdruck P₀ ist wesentlich größer als der Druck, mit dem die einzelnen Hohlkörper 7 gereinigt werden. Er liegt beispielsweise in der Größenordnung von 1 bis 1,5 bar. Ist der Strömungsweg im wesentlichen offen, so wird an der jeweiligen Strömungsverengung 4 ein Druckabfall auftreten, so daß am strömungsseitigen Ausgang der Strömungsverengung 4 ein geringerer Druck P₁ ansteht, der beispielsweise bei 300 mbar liegt. Dieser Druck P₁ beaufschlagt den jeweiligen Sensor 5. Der Sensor 5 ist mit einer Speicherfunktion ausgestattet, die speichert, ob der gemessene Druck P₁ einen vorbestimmten Wert überschritten hat. Dieser Wert kann beispielsweise bei 600 mbar liegen und ist signifikant dafür, daß der jeweilige Hohlkörper ganz oder teilweise verstopft ist. Der Drucksensor speichert, ob dieser erhöhte Wert einmal überschritten wurde und gibt dann ein dauerhaftes Signal ab, das auch nach erneutem Unterschreiten dieses erhöhten Druckwertes erhalten bleibt. Damit kann nach Beendigung des Reinigungsvorganges an den einzelnen Drucksensoren abgelesen werden, ob dort der erhöhte Druckwert aufgetreten ist. Die zugeordneten Hohlkörper können dann aussortiert werden, während die übrigen Hohlkörper als einwandfrei gereinigt gelten.

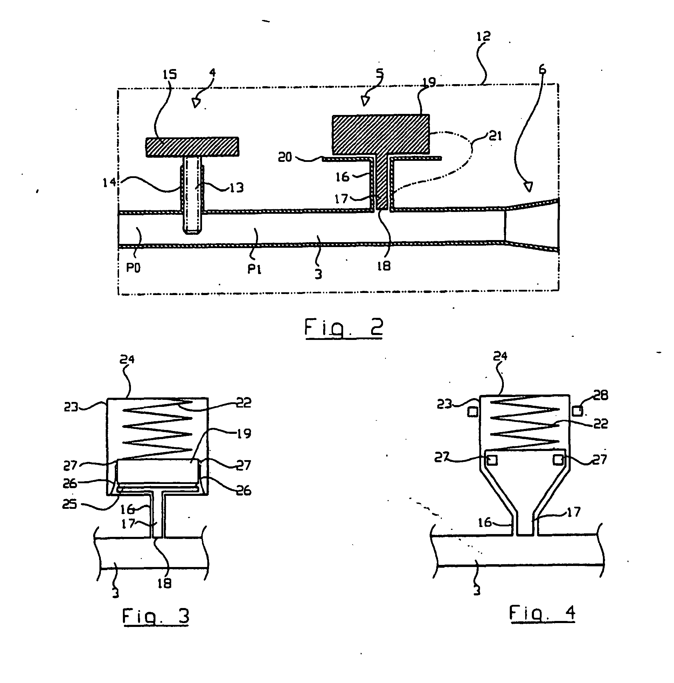

Fig. 2 zeigt ein bevorzugtes Ausführungsbeispiel eines Modules 12 mit einer Strömungsverengung bzw. Düse 4, einem Drucksensor 5 mit Speicherfunktion und einer Kupplung 6. Generell können Drucksensoren der hier verwendeten Art auf die verschiedenste Weise realisiert werden, beispielsweise elektromechanische Sensoren mit elektronischer Speicherung, die allerdings teuer und empfindlich gegen Hitze und Feuchtigkeit sind sowie eine Stromversorgung benötigen, über Sensoren mit mechanischer Haltefunktion (Fig. 3) oder magnetischer Haltefunktion (Fig. 4). Von besonderem Interesse sind hierbei Sensoren, die die Speicherfunktion sowie die Funktion eines Sicherheitsventils bezüglich des maximalen Drucks, der auf den Hohlkörper gegeben wird, eindeutig begrenzt, verknüpfen. Außerdem sollte die Ausführung des Sensors möglichst preiswert, robust gegen Temperatur- und Feuchtigkeitseinwirkung sowie einfach an verschiedene Eingangs- und Reinigungsdrücke P₀ und P₁ adaptierbar sein.

Ein diese Forderung erfüllendes Modul zeigt Fig. 2. Die Strömungsverengung 4 ist als Gewindeschraube 13 ausgeführt, die in eine Gewindehülse 14, die an den Wasserkanal 3 angeschlossen ist, einschraubbar ist und damit in das Innere des Wasserkanals 13 hineinragt. Ein von Hand betätigbarer Drehgriff 15 erlaubt das Ein- und Ausdrehen der Schraube 13. Hiermit kann der Druck P₁ schnell und flexibel den jeweiligen Anforderungen, die sich aus der Dimensionierung der einzelnen Lumen der Hohlkörper 7 ergeben, angepaßt werden.

Eine sehr einfache Ausführung eines Drucksensors 5 besteht aus einer an den Wasserkanal angeschlossenen Hülse 16 und einem darin gleitend verschieblichen Kolben 17, dessen Stirnfläche 18 mit dem Druck P₁ im Wasserkanal 3 beaufschlagt wird. Außerhalb der Hülse 16 ist an den Kolben 17 ein Gewicht 19 angebracht, dessen Durchmesser größer ist als der Durchmesser der Hülse 16 und das den Kolben 17 mit seinem Eigengewicht belastet. Selbstverständlich können der Kolben 17 und das Gewicht 19 einstückig ausgebildet sein. An der Hülse 16 kann eine Scheibe 20 angebracht sein, die als Auflage für das Gewicht 19 dient. Das Gewicht 19 ist durch eine flexible Halterung 21, die beispielsweise ein Kunststoffaden, eine Metallkette oder sonstiges sein kann, an der Hülse oder dem Wasserkanal 3 befestigt und so gegen Verlieren gesichert.

Steigt der Druck P₁ im Wasserkanal an, so werden der Kolben 17 und das Gewicht 19 entgegen der Schwerkraft längs der Hülse 16 vom Wasserkanal 3 fortgedrückt. Erreicht der Druck einen vorbestimmten Grenzwert, so wird der Kolben 17 vollständig aus der Hülse 16 herausgedrückt und die Hülse 16 wirkt damit als Öffnung des Wasserkanals 13 und damit als Überdruckventil. Der Kolben 17 mit Gewicht 19 wird durch die Halterung 21 gegen Verlust gesichert.

Die Speicherfunktion wird also dadurch erfüllt, daß der Kolben 17 mit Gewicht 19 aus der Hülse 16 herausgefallen ist. Dies ist einer Bedienperson sofort ersichtlich und ist so zu interpretieren, daß das Lumen des entsprechenden Hohlkörpers ganz oder teilweise verstopft war, auf alle Fälle also nicht ordnungsgemäß gereinigt wurde.

Die Abdichtung zwischen Hülse 16 und Kolben 17 ist so zu wählen, daß eine Bewegung des Kolbens 17 nicht gehemmt wird, trotzdem aber nur sehr geringe Mengen an Flüssigkeit entweichen können. Durch Dimensionierung der Durchmesser von Hülse 16 und Kolben 17 sowie des Gewichtes 19 kann ein sehr kompakter und einfacher Drucksensor realisiert werden, der vorzugsweise aus Edelstahl besteht und für die hier interessierenden Drücke in der Größenordnung von 100 mbar bis 1 bar ausgelegt werden kann. Die Ansprechempfindlichkeit des Sensors ist durch das Gesamtgewicht des Gewichtes 19 und des Kolbens 17 und die Stirnfläche 18 gegeben.

Fig. 3 zeigt ein Ausführungsbeispiel eines Drucksensors, der ebenfalls die Hülse 16, den Kolben 17 und das Gewicht 19 aufweist, wobei das Gewicht optional auch noch durch eine Feder 22 belastet sein kann. Das Gewicht ist hierbei in einem Gehäuse 23 untergebracht, das an seiner Oberseite ein Sichtfenster 24 aus flexiblem Material aufweist. Das Gewicht hat an seinem radialen Außenumfang eine Nut 25. Im Gehäuse ist mindestens eine radial nach innen gegen das Gewicht vorgespannte Feder 26 angeordnet, die eine Rastnase 27 aufweist. Sind der Kolben 17 und das Gewicht 19 so weit angehoben, daß die Rastnase 27 in die Nut 25 einrastet, so bleibt diese Position von Kolben und Gewicht gespeichert, auch wenn der Druck im Wasserkanal 3 wieder abfällt. Am Sichtfenster 24 ist dann zu erkennen, daß der Kolben 17 und das Gewicht 19 in ihrer oberen Grenzlage sind, also der entsprechende Druckwert überschritten wurde. Da das Sichtfenster flexibel ist, kann das Gewicht z.B. von Hand wieder nach unten in Richtung zum Wasserkanal 3 in seine normale Meßstellung zurückgedrückt werden.

Im Ausführungsbeispiel der Fig. 4 wird die Speicherfunktion durch Magnete 28 und 29 realisiert, die einerseits am Kolben und andererseits am Gehäuse 23 angebracht sind. Sobald der Kolben 17 so weit hochgedrückt ist, daß die Magnete 28 und 29 in etwa fluchten, hält die Magnetkraft den Kolben in der erreichten Stellung, auch wenn der Druck in dem Wasserkanal 3 wieder absinkt. Die Magnetkraft ist dabei so stark, daß auch das Eigengewicht des Kolbens 17 und ggf. auch die Kraft der Feder 22 nicht ausreichen, diese Kraft zu überwinden. Auch hier ist die Oberseite des Gehäuses 22 mit einem durchsichtigen, flexiblen Fenster 24 versehen, durch welches einerseits die Position des Kolbens 17 abgelesen werden kann und andererseits der Kolben auch wieder aus der "Speicherstellung" gelöst und in die Arbeitsstellung gedrückt werden kann.

## Patentansprüche

1. Vorrichtung zum Reinigen und/oder Desinfizieren von Hohlkörpern (7), insbesondere von medizinischen Schläuchen und Kathetern, mit mindestens einer an einen Wasserkanal (3) angeschlossenen Kupplung (6) zur Verbindung des Wasserkanales (3) mit dem Hohlkörper (7), wobei in Strömungsrichtung vor der Kupplung (6) eine an den Wasserkanal (3) angeschlossene Strömungsverengung (4) und ein Drucksensor (5) zum Erfassen und Speichern von in dem Hohlkörper (7) entstehenden Druck angeordnet sind, und der Drucksensor (5) eine an den Wasserkanal (3) angeschlossene Hülse (16) und einen in der Hülse (16) verschieblichen Kolben (17) aufweist.

2. Vorrichtung nach Anspruch 1, **dadurch gekennzeichnet, daß** der Drucksensor (5) so ausgebildet ist, daß er dauerhaft ablesbar speichert, ob der erfaßte Druck einen vorbestimmten Wert überschritten hat.

3. Vorrichtung nach Anspruch 2, **dadurch gekennzeichnet,**
**daß** der Kolben (17) durch sein Eigengewicht und ggf. ein zusätzliches Gewicht (19) in Richtung zum Wasserkanal (3) gedrückt wird und bei dem vorbestimmten Druck vollständig aus der Hülse (16) herausgedrückt wird.

4. Vorrichtung nach Anspruch 3, **dadurch gekennzeichnet,**
**daß** der Kolben mit einer flexiblen Halterung (21) an dem Wasserkanal (3) oder der Hülse (16) befestigt ist.

5. Vorrichtung nach Anspruch 4, **dadurch gekennzeichnet,**
**daß** der Kolben (16) durch eine Feder (22) in Richtung zum Wasserkanal (3) gedrückt wird.

6. Vorrichtung nach einem der Ansprüche 2, 4 oder 5, **dadurch gekennzeichnet,**
**daß** am Kolben (17, 19) eine Rastnut (25) angebracht ist und an einem Gehäuse (23) eine Feder (26), die bei Erreichen einer vorbestimmten Grenzstellung in die Rastnut (25) einrastet.

7. Vorrichtung nach einem der Ansprüche 2, 4 oder 5, **dadurch gekennzeichnet,**
**daß** an dem Kolben mindestens ein Magnet (27) und an einem Gehäuse (23), in welchem der Kolben (17) verschieblich geführt ist, ein Gegenmagnet (28) angebracht ist.

8. Vorrichtung nach einem der Ansprüche 1 bis 7, **dadurch gekennzeichnet,**
**daß** die Strömungsverengung (4) einstellbar ist.

9. Vorrichtung nach Anspruch 8, **dadurch gekennzeichnet,**
**daß** die Strömungsverengung (4) eine in den Wasserkanal (3) einschraubbare Schraube (13) ist.

## Claims

1. Device for cleaning and / or disinfecting of hollow bodies (7), in particular of medical tubes and catheters, having at least one coupling (6) connected to a water channel (3), for connecting the water channel (3) to the hollow body (7), wherein upstream of the coupling (6) a flow constriction (4) and a pressure sensor (5) to capture and store the pressure of the hollow body (7) are connected to the water channel (3), and the pressure sensor (5) comprises a sleeve (16) connected to the water channel (3) and a piston (17) movable within the sleeve (16) .

2. Device according to claim 1, **characterized in that** the pressure sensor (5) is designed such that it permanently stores to read, whether the sensed pressure did exceed a predetermined value.

3. Device according to claim 2, **characterized in that** the piston (17) is pressed by its own weight and, as the case may be, any additional weight (19), in the direction of the water channel (3) and is pushed completely out of the sleeve (16) at the predetermined pressure.

4. Device according to claim 3, **characterized in that** the piston is fastened to the water channel (3) or to the sleeve (16) by a flexible holder (21).

5. Device according to claim 4, **characterized in that**, that the piston (16) is pressed by a spring (22) in the direction of the water channel (3).

6. Device according to claim 2, 4 or 5, **characterized in that**
the piston (17, 19) has a latching groove (25) and a housing (23) has a spring (26) that snaps into the latching groove (25) on reaching a predetermined limit position.

7. Device according to claim 2, 4 or 5, **characterized in that**
at least one magnet is attached to the piston (27) and a counter-magnet (28) is attached to a housing (23), in which the piston (17) is guided for displacement,

8. Device according to claims 1 to 7, **characterized in that**
the flow constriction (4) is adjustable.

9. Device according to claim 8, **characterized in that** the flow constriction (4) is a screw (13) which can be screwed into the water channel (3).

## Revendications

1. Dispositif de nettoyage et/ou de désinfection de corps creux (7), en particulier, de tubes médicaux et de cathéters, comportant au moins un couplage (6) raccordé à au moins un canal d'eau (3) pour relier le canal d'eau (3) au corps creux (7), dans lequel on prévoit un rétrécissement d'écoulement (4) raccordé, dans le sens de l'écoulement, au canal d'eau (3) avant le couplage (6), et un capteur de pression (5) pour saisir et enregistrer la pression observée dans le corps creux (7), et dans lequel le capteur de pression (5) présente une gaine (16) raccordée au canal d'eau (3) et un piston (17) pouvant être poussé dans la gaine (16).

2. Dispositif selon la revendication 1, **caractérisé en ce que** le capteur de pression (5) est conçu de telle sorte qu'il enregistre, de façon durablement lisible, si la pression saisie a dépassé une valeur prédéterminée.

3. Dispositif selon la revendication 2, **caractérisé en ce**
**que** le piston (17) est poussé par son propre poids et éventuellement par un poids supplémentaire (19) dans le sens du canal d'eau (3) et sort complètement de la gaine (16) par la pression prédéterminée.

4. Dispositif selon la revendication 3, **caractérisé en ce**
**que** le piston est fixé avec un support flexible (21) au canal d'eau (3) ou à la gaine (16).

5. Dispositif selon la revendication 4, **caractérisé en ce**
**que** le piston (16) est poussé par un ressort (22) dans le sens du canal d'eau (3).

6. Dispositif selon l'une des revendications 2, 4 ou 5, **caractérisé en ce**
**qu'**une encoche d'arrêt (25) est aménagée sur le piston (17, 19), et sur un boîtier (23) se trouve un ressort (26) qui est encliqueté lorsqu'une position limite prédéterminée est atteinte dans l'encoche d'arrêt (25).

7. Dispositif selon l'une des revendications 2, 4 ou 5, **caractérisé en ce**
**que** sur le piston on prévoit au moins un aimant (27), et sur le boîtier (23) dans lequel le piston (17) peut être engagé de façon déplaçable on prévoit un contre-aimant (28).

8. Dispositif selon l'une des revendications 1 à 7, **caractérisé en ce**
**que** le rétrécissement d'écoulement (4) est réglable.

9. Dispositif selon la revendication 8, **caractérisé en ce**
**que** le rétrécissement d'écoulement (4) est une vis (13) pouvant être vissée dans le canal d'eau (3).
